(19) Europäisches Patentamt
European Patent Office
Office européen des brevets

(11) **EP 2 744 502 B1**

(12) **EUROPEAN PATENT SPECIFICATION**

(45) Date of publication and mention
of the grant of the patent:
**30.03.2016 Bulletin 2016/13**

(21) Application number: **12823628.8**

(22) Date of filing: **16.08.2012**

(51) Int Cl.:
*A61K 31/708* (2006.01)    *A61K 31/717* (2006.01)
*A61K 31/7088* (2006.01)    *A61K 31/713* (2006.01)

(86) International application number:
**PCT/US2012/051161**

(87) International publication number:
**WO 2013/025918 (21.02.2013 Gazette 2013/08)**

(54) **METHODS USEFUL IN THE PREVENTION OF HYPOXIC INJURY**

VERFAHREN ZUR PRÄVENTION VON HYPOXISCHEN LÄSIONEN

MÉTHODES UTILES DANS LA PRÉVENTION D'UNE LÉSION HYPOXIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.08.2011 US 201161524696 P**

(43) Date of publication of application:
**25.06.2014 Bulletin 2014/26**

(73) Proprietor: **Oregon Health & Science University
Portland, OR 97239 (US)**

(72) Inventors:
• **BAHJAT, Frances Rena**
**Portland, Oregon 97201 (US)**
• **STENZEL-POORE, Mary**
**Lake Oswego, Oregon 97035 (US)**

(74) Representative: **Atkinson, Jennifer
Barker Brettell LLP
100 Hagley Road
Edgbaston
Birmingham B16 8QQ (GB)**

(56) References cited:
**WO-A1-2005/102278    US-A- 4 349 538
US-A1- 2009 087 454**

• **RAFFAELLA GESUETE ET AL: "Poly-ICLC
preconditioning protects the blood-brain barrier
against ischemic injury in vitro through type I
interferon signaling", JOURNAL OF
NEUROCHEMISTRY, vol. 123, 11 October 2012
(2012-10-11), pages 75-85, XP055159487, ISSN:
0022-3042, DOI:
10.1111/j.1471-4159.2012.07946.x**
• **Tulin Alkan ET AL: "Neuroproctective effects of
ischemic tolerance (preconditioning) and
postconditioning", Turkish neurosurgery, 1
October 2009 (2009-10-01), pages 406-412,
XP055159491, Turkey Retrieved from the
Internet:
URL:http://www.ncbi.nlm.nih.gov/pubmed/198
47763**
• **HIDEHO OKADA: "Brain Tumor Immunotherapy
with Type-1 Polarizing Strategies", ANNALS OF
THE NEW YORK ACADEMY OF SCIENCES, vol.
1174, no. 1, 1 September 2009 (2009-09-01), pages
18-23, XP55159023, ISSN: 0077-8923, DOI:
10.1111/j.1749-6632.2009.04932.x**
• **S. FUKUDA ET AL: "Cerebral protection",
BRITISH JOURNAL OF ANAESTHESIA, vol. 99,
no. 1, 1 July 2007 (2007-07-01), pages 10-17,
XP055159018, ISSN: 0007-0912, DOI:
10.1093/bja/aem140**

## Description

### FIELD

This disclosure relates to the field of treatment of cytotoxic insults with pharmaceutical compositions and specifically to the treatment of cytotoxic insults such as excitotoxic, ischemic and/or hypoxic events with pharmaceutical compositions comprising poly-ICLC.

### ACKNOWLEDGEMENT OF GOVERNMENT SUPPORT

Aspects of this invention were made with United States government support pursuant to grant number 1 R01 NS062381-01A1 awarded by the National Institutes of Health. The United States government has certain rights in the invention.

### BACKGROUND

Patients undergoing cardiac surgery run the risk of suffering from ischemic injury. Ischemic injuries resulting from cardiac surgery commonly affect the brain, kidneys, and lungs (See: Barber PA et al, Stroke 39, 1427-1433 (2008); Fukada J et al, Surgery Today 34, 11-15 (2004); and Mehta RH et al, The American Journal of Cardiology 106, 1728-1734 (2010), all of which are incorporated by reference herein). Approximately 50% of patients who undergo cardiac surgery develop one or more new ischemic lesions in the brain. One study even shows a 63% chance of cognitive decline following cardiac surgery (Barber PA *et al,* (2008) *supra).* These complications are known to increase post-operative decline, and mortality. Even though there is a need for preventative treatments of ischemic injuries, few treatments exist.

One such treatment takes advantage of the concept of ischemic tolerance whereby brief exposure to a harmful stimulus, such as ischemia, volatile anesthetics, hypothermia, hypoxia or inflammation, given prior to an ischemic challenge provides protection against lethal ischemia (See Dirnagl U et al, Trends in Neuroscience 26, 248-254 (2003) incorporated by reference herein). However these treatments involve methods such as blocking blood flow to the brain for 15 minutes and would therefore be unlikely to be appropriate for therapy.

Specifically, preconditioning with lipopolysaccharide (LPS) confers robust protection against ischemic injury (See Heemann U et a/, American Journal of Pathology 156, 287-293 (2000); Hua F et al, Journal of Neuroimmunology 199, 75-82 (2008); Rosenzweig HL et al, J Cereb Blood Flow & Metab 27,1663-1674 (2007); Stevens SL et al, J Cereb Blood Flow Metab 28, 1040-1047 (2008); Tasaki et al, Brain Research 748, 267-270 (1997) all of which are incorporated by reference herein.) However, treatment with LPS is not a viable option for therapeutic consideration due to its known harmful side effects including serum sickness and sepsis.

Clearly there is a need for treatments of ischemic injury, especially treatments that are not harmful in and of themselves and/or have such deleterious side effects. More particularly, there is a need for a treatment of ischemic injury that lacks the proinflammatory effects of LPS.

### SUMMARY

The invention relates to composition for use in methods as stated in the claims.

Methods of treating cellular and tissue damage caused by cytotoxic insult are disclosed. Such methods provide a treatment that is as effective as methods currently in use, but significantly safer than those methods currently in use. This is evidenced by the fact that the disclosed method results in the expression of a lower level of inflammatory cytokines relative to treatment with other agents such as LPS. However, the disclosed methods are at least as effective as, if not more effective than, treatment with LPS.

It is disclosed herein that compositions comprising poly-ICLC that, when administered to a subject at risk of developing a hypoxic injury prior to the hypoxic injury, will protect the subject from the hypoxic injury. The compositions comprise poly-ICLC in a pharmaceutically acceptable carrier and are formulated to deliver a systemic dose of poly-ICLC to the subject.

Also disclosed herein are methods of protecting subjects at risk of developing a hypoxic injury from the hypoxic injury. The method comprises systemically administering a pharmaceutical composition comprising poly-ICLC in a pharmaceutically acceptable carrier to the subject.

The disclosed compositions and methods are shown herein to protect the brain from the effects of hypoxic injury in an accepted animal model for hypoxic injury. The disclosed compositions and methods work in part by maintaining the structure of the blood-brain barrier.

**DESCRIPTION OF THE DRAWINGS**

Figure 1A is a bar graph depicting the results of an experiment in which mixed cortical cultures were preconditioned with poly-ICLC (1 to 1,000 ng/ml as indicated) 24 hours before 3-hour oxygen glucose deprivation (OGD). A representative example of three independent experiments is shown. Bar graph values are group means with error bars representing the standard error of the mean; ** - $P < 0.01$, *** - $P < 0.001$ versus vehicle control by analysis of variance (ANOVA) and Bonferroni's post hoc test.

Figure 1B is a set of images depicting representative sections of 2, 3, 5, triphenyltetrazolium chloride-stained brain sections from vehicle-treated (top) and poly-ICLC-treated (bottom) animals.

Figure 1C is a set of two box and whisker (min/max) plots of collective infarct volume of mice treated with the indicated dose of poly-ICLC (left graph) or LPS (right graph).

Figure 1D is a set of two bar graphs showing the general neurological score (left graph) and the focal neurological score (right graph) at the indicated doses of poly-ICLC. Values are mean with the error bars representing the standard error of the mean.

Figure 1E is a bar graph showing the results of the corner test score for mice treated with vehicle and poly-ICLC. The shaded horizontal bar shows the corner test score range for untreated mice.

Figure 2A is a bar graph showing the plasma concentration of IL-1$\beta$ after administration with vehicle, LPS (1 mg/kg), or poly-ICLC (1.6 mg/kg), n=3 mice pretreatment. IL-1$\beta$ concentrations were assessed by custom multiplex ELISA. ** - $P < 0.01$ by ANOVA and Bonferroni's *post hoc* test.

Figure 2B is a bar graph showing the plasma concentration of IL-6 after administration with vehicle, LPS (1 mg/kg), or poly-ICLC (1.6 mg/kg), n=3 mice per treatment. IL-6 concentrations were assessed by cytometric bead assay. ** - $P < 0.01$ by ANOVA and Bonferroni's *post hoc* test.

Figure 2C is a bar graph showing the plasma concentration of IL-12 after administration with vehicle, LPS (1 mg/kg), or poly-ICLC (1.6 mg/kg), n=3 mice per treatment. IL-12 concentrations were assessed by custom multiplex ELISA. *** - $P < 0.001$ by ANOVA and Bonferroni's *post hoc* test.

Figure 2D is a bar graph showing the plasma concentration of TNF$\alpha$ after administration with vehicle, LPS (1 mg/kg), or poly-ICLC (1.6 mg/kg), n=3 mice per treatment. TNF$\alpha$ concentrations were assessed by cytometric bead assay. ** - $P < 0.01$ by ANOVA and Bonferroni's *post hoc* test.

Figure 2E is a bar graph showing the plasma concentration of IFN$\gamma$ after administration with vehicle, LPS (1 mg/kg), or poly-ICLC (1.6 mg/kg), n=3 mice per treatment. IFN$\gamma$ concentrations were assessed by custom multiplex ELISA. ** - $P < 0.01$ by ANOVA and Bonferroni's *post hoc* test.

Figure 3A is a graphical depiction of the basic experimental design used in Examples 5-10. BMECs and glial cells were co-cultured for 2 days followed by preconditioning with poly-ICLC (10ng/ml to 2$\mu$g/ml). Twenty-four hours later co-cultures were exposed to 5 hours of oxygen-glucose deprivation (OGD).

Figure 3B is a bar graph showing data of the transendothelial electrical resistance (TEER) values for the indicated conditions. Data are reported as mean $\pm$ SEM. ***-indicates $P < 0.001$ vs control, °°-indicates $P < 0.01$ vs cells subjected to OGD.

Figure 3C is a set of two bar graphs showing the endothelial permeability (Pe) for Na fluorescein and albumin as indicated for the indicated conditions. Data are reported as mean $\pm$ SEM. **-indicates $P < 0.01$ vs control, *** - indicates $P < 0.001$ vs controls, °-indicates $P < 0.05$ vs cells subjected to OGD, and °°- indicates $P < 0.01$ vs cells subjected to OGD.

Figure 4A is a bar graph showing the concentration of IFN-$\beta$ protein measured by ELISA in culture medium collected under the indicated conditions. Data are reported as mean $\pm$ SEM. *** - indicates $P < 0.001$ vs controls.

Figure 4B is a bar graph showing the expression of IFN-$\beta$ mRNA in cells collected under the indicated conditions. Data are reported as mean $\pm$ SEM. *** - indicates $P < 0.001$ vs controls.

Figure 5A is a plot showing the TEER results from cultures that include astrocytes and microglia from IFN$\beta$ knockout mice. Data are reported as mean $\pm$ SEM. ** $P<0.01$ and *** $P<0.001$ vs CTR.

Figure 5B is a set of two bar graphs showing Pe values for Na fluorescein and albumin as indicated from cultures that include astrocytes and microglia from IFN$\beta$ knockout mice. ** $P<0.01$ and *** $P<0.001$ vs CTR.

Figure 6A is a set of four panels showing (far left) a depiction of wild-type BMECs in culture with IFNAR knockout glial cells, (second from left) TEER results, (second from right) Pe results using Na fluorescein and (far right) Pe results using albumin from these cultures.

Figure 6B is a set of four panels showing (far left) a depiction of IFNAR knockout BMECs in culture with wild type knockout glial cells, (second from left) TEER results, (second from right) Pe results using Na fluorescein and (far right) Pe results using albumin from these cultures.

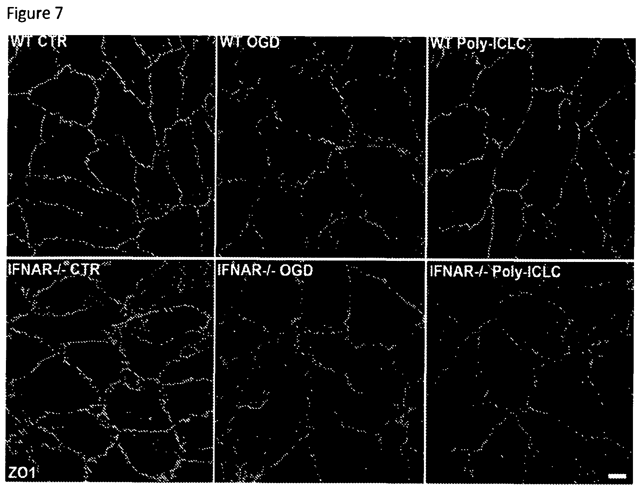

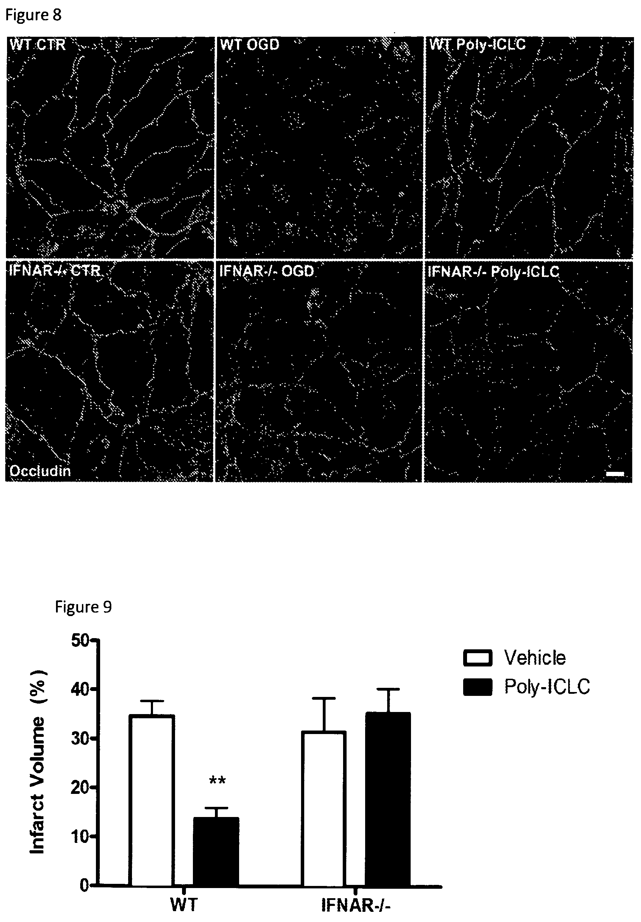

Figure 7 is an image of BMEC's from wild type or IFNAR knockout mice as indicated, subjected to OGD as indicated, treated with poly-ICLC as indicated and stained with fluorescent antibody specific for ZO-1. Scale bar = 10 $\mu$m. Figure 8 is an image of BMEC's from wild type or IFNAR knockout mice as indicated, subjected to OGD as indicated, treated with poly-ICLC as indicated and stained with fluorescent antibody specific for Occludin. Scale bar = 10 $\mu$m. Figure 9 is a bar graph of the results of wild type and IFNAR knockout mice preconditioned with poly-ICLC prior to MCAO. Infarct size was determined 24 hours following MCAO by TTC staining. Data are reported as mean $\pm$ SEM, **-p < 0.01.

## DETAILED DESCRIPTION

In one embodiment of the invention, poly-ICLC is administered to a subject at risk of developing a hypoxic injury in order to protect that subject from the hypoxic injury.

### Terms

Unless otherwise explained, all technical and scientific terms used herein have the same meaning as commonly understood by one of ordinary skill in the art to which this disclosure belongs. Definitions of common terms in molecular biology may be found in Benjamin Lewin, Genes V, published by Oxford University Press, 1994 {ISBN 0-19-854287-9); Kendrew eta/. {eds.), The Encyclopedia of Molecular Biology, published by Blackwell Science Ltd., 1994 {ISBN 0-632-02182-9); and Robert A. Meyers {ed.), Molecular Biology and Biotechnology: a Comprehensive Desk Reference, published by VCH Publishers, Inc., 1995 {ISBN 1-56081-569-8). The singular terms "a," "an," and "the" include plural referents unless context clearly indicates otherwise. Similarly, the word "or" is intended to include "and" unless the context clearly indicates otherwise. The term "plurality" refers to two or more. It is further to be understood that all molecular weight or molecular mass values are approximate, and are provided for description. Although methods and materials similar or equivalent to those described herein can be used in the practice or testing of this disclosure, suitable methods and materials are described herein. The term "comprises" means "includes." The abbreviation, "e.g." is derived from the Latin exempli gratia, and is used herein to indicate a non-limiting example. Thus, the abbreviation "e.g." is synonymous with the term "for example."

A **cytokine** may be any soluble protein (or glycoprotein) involved in the regulation of cellular proliferation and function. A cytoprotective cytokine may be any cytokine that acts to preserve cellular function and prevent (or reduce) death of a cell in response to a stressful or otherwise aversive stimulus. Cytoprotective cytokines include transforming growth factor $\beta$ (TGF-$\beta$), tumor necrosis factor $\alpha$ (TNF $\alpha$), and type I interferons, such as interferon $\beta$ (IFN$\beta$). A neuroprotective cytokine may be any cytoprotective cytokine that acts to preserve cellular function and reduce cell death in neural cells. Cytoprotective cytokines and neuroprotective cytokines include all naturally occurring and artificial cytokines that meet the above description now known or yet to be disclosed.

An **excitotoxic injury** refers to the injury (up to and including death), of cells, neural cells, neural cells of the brain, kidney cells and other cells due to excessive stimulation of cell-surface receptors. Most commonly, excitotoxic injury is mediated through glutamate receptors, for example, by overactivation of N-methyl-d-aspartate (NMDA)-type glutamate receptors, resulting in excessive Ca$^{++}$ influx through the receptor's associated ion channel. Excitotoxic injury is believed to play a role in diverse conditions, including epilepsy, traumatic injury, and Alzheimer's disease.

A state of **hypoxia** may be any physiological or cellular state in which one or more cells are not exposed to oxygen. In a physiological context, the term hypoxia refers to an insufficiency of oxygen at a cellular, tissue or organismal level. Hypoxia can be caused by, for example, the reduction in partial pressure of oxygen (in the blood or in a tissue), inadequate oxygen transport (for example, due to a failure of oxygenated blood to reach a target tissue or cell), inadequate respiratory function (for example due to bronchial or tracheal blockage or choking) or the inability of the tissues to use oxygen. An infarct may be any kind of necrotic, apoptotic or other cell, tissue or organismal death resulting from any insufficiency of oxygen (hypoxia).

Hypoxia may be the result of **ischemia.** Ischemia may be any reduction in the flow of oxygenated blood to a tissue or organ. Similarly, an ischemic event may be any event, action, process, injury, or other disruption that results in decreased blood flow to a cell, collection or group of cells, tissue, or organ. Examples of ischemic events include vasoconstriction, thrombosis and embolism.

A **neural cell** may be any cell derived from a lineage that originates with a neural stem cell and includes a mature neuron. Thus, the term neural cell includes neurons (nerve cells) as well as their progenitors regardless of their stage of differentiation. In the context of an adult brain, neural cells are predominantly differentiated neurons. In one example, neural cells include hippocampal neurons and cortical neurons. In contrast, a **non-neural** cell may be any cell derived from any lineage other than a neural cell lineage. That is, it may be any cell does not terminally differentiate into a mature neuron. Some non-neural cells make up part of the central nervous system (CNS). Examples of non-neural cells in the CNS include cells of the brain (such as glial cells and immune system cells, such as B cells, dendritic cells, macrophages and microglia). Other non-neural cells may make up a tissue or organ outside the CNS, such as cardiac, skeletal or smooth muscle (a muscle cell), lung, intestine, liver (a hepatic cell) or kidney (a renal cell) and so forth. Non-neural cells further include cells of the immune system (such as a dendritic cell, lymphocyte, macrophage or a microglial cell).

A **preconditioning dose** of a compound is an effective dose of an active compound (such as poly-ICLC) that protects a cell against future injury or death due to an excitotoxic, ischemic or hypoxic event. The preconditioning dose of a compound or composition varies from compound to compound and between species. A suitable preconditioning dose for any compound or composition can be determined empirically.

**Prophylactic** treatment of a cellular or physiological condition refers to the treatment of a subject prior to the full manifestation of the condition or any disease, event, action, process, injury, or other disruption that causes or may cause the condition for the purpose of preventing or reducing the symptoms, signs or consequences of the condition. Thus, in the context of the present disclosure, prophylactic treatment of an excitotoxic injury or hypoxia refers to the treatment of a subject prior to the occurrence of an excitotoxic or hypoxic event (that is, prior to a first excitotoxic or hypoxic event, or prior to a subsequent excitotoxic or hypoxic event, or prior to the completion or culmination of an ongoing or recurrent excitotoxic or hypoxic event) or prior to the completion of the natural consequences and/or sequelae of the event. For example, a subject may be treated prophylactically using a composition that comprises poly-ICLC prior to cardiac surgery. Prophylactic treatment may in the case of cytotoxic insult may also be referred to as preconditioning.

The term **"protect"** with respect to an excitotoxic or hypoxic event refers to the ability of the composition or treatment regimen to prevent, reduce in severity, or otherwise lessen the effects of an excitotoxic or hypoxic event, including a hypoxic event resulting from ischemia at a cellular, tissue, or organismal level.

A subject may be considered at **risk of hypoxic injury** if there is an increased probability that the subject will undergo an event resulting in hypoxic injury relative to the general population. Accordingly, risk is a statistical concept based on empirical and/or actuarial data. Commonly, risk can be correlated with one or more indicators, such as symptoms, signs, characteristics, properties, occurrences, events or undertakings, of a subject. For example, with respect to hypoxic injury in the brain resulting from ischemia, indicators include but are not limited to high blood pressure (hypertension), atrial fibrillation, transient ischemic events, prior stroke, diabetes, high cholesterol, angina pectoris, and heart disease.

Additional risk indicators for hypoxic injury include surgery, especially cardiovascular surgeries, such as endarterectomy, pulmonary bypass surgery or coronary artery bypass surgery. Additional risk factors or indicators include non-medical activities, such as motorcycle riding, contact sports and combat. Other risk factors are discussed herein, and yet more can be recognized by those of ordinary skill.

A **stroke** may be any interruption of blood flow to any part of the brain. A stroke can be due to an ischemic event (for example, occlusion of a blood vessel due to a thrombus or an embolism) or hemorrhage (for example of a cerebral blood vessel). Either of these events may result in hypoxia.

A **subject** may be any living multi-cellular vertebrate organism including both human and veterinary subjects such as mammals. A subject also includes livestock and companion animals.

The term **"medicament"** is used interchangeably with the term **"pharmaceutical composition."** Such compositions are formulated for administration to human and/or animal (veterinary) subjects, and can include one or more active component (such as poly-ICLC), as well as one or more additional components to facilitate administration to a subject, for the therapeutic or prophylactic treatment (prevention or reduction) of a condition or disorder. The additional components can include pharmaceutically acceptable carriers, buffers or excipients. Pharmaceutically acceptable carriers, buffers and so forth, are well known in the art, and are described, e.g., in Remingtons Pharmaceutical Sciences, 19th Ed., Mack Publishing Company, Easton, Pennsylvania, 1995.

**Systemic administration** may be any administration that results in the composition having an effect on cells and/or tissues at one or more sites at a distance to the site of administration, including cells and/or tissues of an organ or body part that is not the organ or body part into which the composition is directly administered as well as an effect at or near the site of administration. Intravenous administration is one method of systemic administration of a composition. Additionally, a composition can be systemically administered by introducing the composition into a site that will ultimately reach the circulatory system of the organism. The composition may reach the circulatory system by any process including diffusion or any of a number of active transport processes. As a result, intranasal, oral, transdermal, subcutaneous, intramuscular, intravenous, intrathecal and intraperitoneal routes are all considered systemic administration of the composition. Systemic administration may be distinguished from one or more administration routes that result in a composition being retained in close proximity (for example, within the same tissue or organ) to the site of introduction.

A **treatment** may be any action that is intended to treat or reduce the symptoms, signs or consequences of a condition or any disease, event, action, process, injury, or other disruption that causes or may cause the condition to occur. Thus, in the context of the present disclosure, treatment includes protecting a subject at risk of developing a hypoxic injury. However, one skilled in the art will appreciate that a treatment, such as a protective treatment need not completely protect from the development of the symptoms, signs or consequences of the hypoxic injury in order for the protection to be considered effective. For example, a notable reduction in the adverse effects associated with a hypoxic injury can be sufficient. For example, a therapeutic composition can decrease the sign or symptom by a desired amount, for example by at least 20%, at least 50%, at least 80%, at least 90%, at least 95%, at least 98%, or even at least 100%, as compared to the sign or symptom in the absence of the composition that comprises poly-ICLC. Similarly, an effective dose or amount of a composition comprising poly-ICLC need not be a dose that completely ameliorates the symptoms, signs, or consequences of the condition.

An **effective amount or dose** of poly-ICLC is the amount or dose of poly-ICLC included in a composition, for example in a pharmaceutical composition, that is an amount or dose determined to protect a subject from the adverse effects of hypoxic injury. Administration of a composition comprising an effective dose of poly-ICLC to a subject at risk of developing a hypoxic injury (e.g., due to ischemia), can be used to protect the subject from the results of the hypoxic injury.

Unless otherwise explained, all technical and scientific terms used herein have the same meaning as commonly understood by one of ordinary skill in the art to which this disclosure belongs.

### Poly-ICLC

Polyinosinic polycytadylic acid (interchangeably called In.Cn., poly-IC or poly I:C among others) is a synthetic version of double stranded RNA that induces an innate immune response. Poly-IC is comprised of a polymer of inosinic acid and a polymer of cytidylic acid hydrogen bonded to one another in a similar manner to nucleic acid strands. See Field et al, PNAS USA 58, 1004-1010 (1967), Levy et al, PNAS USA 62, 357-361 (1969), both of which are incorporated by reference herein. While designed to induce an immune response, Poly-IC is readily hydrolyzed by serum nucleases, thereby decreasing its activity.

Poly-ICLC (interchangeably known as Hiltonol® or poly-IC:LC, among others) is a high molecular weight derivative of poly-IC stabilized with poly L-lysine and carboxymethylcellulose (CMC) that have been added to improve the pharmacokinetic properties of poly-IC. Poly-ICLC therefore has a formula of In.Cn-poly-1-lysine-carboxymethylcellulose. See U.S. Patent Number 4,349,538, filed 18 Nov 1980, incorporated by reference herein. Carboxymethylcellulose is a negatively charged (at neutral pH), hydrophilic material used to maintain the solubility of the complex. Poly-ICLC is more resistant to nucleases than poly-IC with a 27,000 KDa or larger complex of poly-ICLC being particularly resistant to nucleases (*Ibid*).

Poly-ICLC has shown great clinical promise in humans for indications such as multiple sclerosis, cancer, and viral infections (See: Markosian M and Hyde RM, Antiviral Chemistry and Chemotherapy 16, 91-102 (2005); Rosenfeld MR et al, Neuro-oncology 12, 1071-1077 (2010), and Salazar WO 2005/102278, published 03 Nov 2005; all of which are incorporated by reference herein).

### Pharmaceutical compositions that include poly-ICLC as an ingredient

A pharmaceutical composition may be any composition of matter formulated in such a way that it may be administered to a subject, for example a subject at risk of the adverse effects of a cytotoxic insult, such as excitotoxic, ischemic, or hypoxic events (or combinations thereof) or a subject suffering from the adverse effects of cytotoxic insult. The amount of poly-ICLC included in the pharmaceutical composition is an amount determined to treat or protect a subject from the adverse effects of cytotoxic insult (referred to herein as an effective amount or dose).

A pharmaceutical composition may also be termed a medicament and the terms are used interchangeably. A pharmaceutical composition can include one or more active components (such as poly-ICLC). The poly-ICLC may be formulated in a range of molecular weights, such that in some pharmaceutical compositions, the molecular weight of the poly-ICLC may be at least about 10,000 daltons, for example more than about 10,000 daltons. In other pharmaceutical compositions, it may be more than about 30,000 daltons. In particular embodiments, the poly-ICLC may be at least about 11,000 daltons, at least about 12,000 daltons, at least about 13,000 daltons, at least about 14,000 daltons, at least about 15,000 daltons, at least about 16,000 daltons, at least about 17,000 daltons, at least about 18,000 daltons, at least about 19,000 daltons, at least about 20,000 daltons, at least about 21,000 daltons, at least about 22,000 daltons, at least about 23,000 daltons, at least about 24,000 daltons, at least about 25,000 daltons, at least about 26,000 daltons, at least about 27,000 daltons, at least about 28,000 daltons, at least about 29,000 daltons, at least about 30,000 daltons, at least about 31,000 daltons, at least about 32,000 daltons, at least about 33,000 daltons, at least about 34,000 daltons, at least about 35,000 daltons, at least about 36,000 daltons, at least about 37,000 daltons, at least about 38,000 daltons, at least about 39,000 daltons, at least about 40,000 daltons, at least about 45,000 daltons, at least about 50,000 daltons, at least about 55,000 daltons, at least about 60,000 daltons, at least about 65,000 daltons, at least about 70,000 daltons, at least about 75,000 daltons, at least about 80,000 daltons, at least about 85,000 daltons, at least about 90,000 daltons, at least about 95,000 daltons, at least about 100,000 daltons, or more. In other embodiments, the poly-ICLC may be about 10,000 to about 100,000 daltons, about 10,000 to about 80,000 daltons, about 10,000 to about 60,000 daltons, about 10,000 to about 40,000 daltons, about 10,000 to about 35,000 daltons, about 10,000 to about 34,000 daltons, about 10,000 to about 33,000 daltons, about 10,000 to about 32,000 daltons, about 10,000 to about 31,000 daltons, about 10,000 to about 30,000 daltons, about 20,000 to about 30,000 daltons, about 20,000 to about 35,000 daltons, about 20,000 to about 40,000 daltons, about 20,000 to about 50,000 daltons, about 27,000 to about 31,000 daltons, about 30,000 to about 32,000 daltons, about 30,000 to about 33,000 daltons, about 30,000 to about 34,000 daltons, about 30,000 to about 35,000 daltons, about 30,000 to about 40,000 daltons, about 30,000 to about 50,000 daltons, about 30,000 to about 60,000 daltons, about 30,000 to about 70,000 daltons, about 30,000 to about 80,000 daltons, about 30,000 to about 90,000 daltons, about 30,000 to about 100,000 daltons.

The pharmaceutical composition may also include one or more additional components that facilitate administration. Examples of additional components include pharmaceutically acceptable carriers, buffers or excipients. Pharmaceutically acceptable carriers, buffers and so forth, are well known in the art, and are described, *e.g.*, in Remington's Pharmaceutical Sciences, 19th Ed., Mack Publishing Company, Easton, Pa., 1995. The pharmaceutical composition can contain appropriate salts and buffers to render the components of the composition stable and facilitate administration to a subject. Further, formulating a pharmaceutical composition to be used via a particular route of administration or to generate a particular size or timing of dose (instantaneous or timed release) is similarly well known in the art. A pharmaceutical composition may be used for the therapeutic or prophylactic treatment of a condition or disorder.

In general, the nature of the carrier will depend on the particular mode of administration being employed. For instance, parenteral formulations usually include injectable fluids that include pharmaceutically and physiologically acceptable fluids such as water, physiological saline, balanced salt solutions, aqueous dextrose, glycerol or the like as a vehicle. For solid compositions (e.g., powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example, sodium acetate or sorbitan monolaurate.

Formulations suitable for topical administration include pastilles including the active ingredient in an inert base, such as gelatin and glycerin, or sucrose and acacia, as well as creams, emulsions, and gels containing, in addition to the active ingredient, such carriers as are known in the art. Furthermore, transdermal patches can be prepared using methods known in the art. For example, the pharmaceutical compositions can include one or more of a stabilizing detergent, a micelle forming agent, and/or an oil. Suitable stabilizing detergents, micelle-forming agents, and oils are detailed in U.S. Pat. Nos. 5,585,103; 5,709,860; 5,270,202; and 5,695,770. A stabilizing detergent is any detergent that allows the components of the emulsion to remain as a stable emulsion. Such detergents include polysorbate 80 (TWEEN®) (Sorbitan-mono-9-octadecenoate-polyoxy-1,2-ethanediyl; manufactured by ICI Americas, Wilmington, Del.), TWEEN 40™, TWEEN 20™, TWEEN 60™ Zwittergent M3-12™ , TEEPOL HB7™ and SPAN 85™. These detergents can be provided in an amount of approximately 0.05 to 0.5%, such as at about 0.2%.

A micelle forming agent is an agent which is able to stabilize the emulsion formed with the other components such that a micelle-like structure is formed. Such agents generally cause some irritation at the site of injection in order to recruit macrophages to enhance the cellular response. Examples of such agents include polymer surfactants described by, e.g., Schmolka, J. Am. Oil. Chem. Soc. 54: 110, 1977, and Hunter et al., J. Immunol. 129: 1244,1981, and such agents as PLURONIC™ L62LF, LIOI, and L64, PEG1000 and TETRONIC™ 1501, 150R1, 701, 901,1301, and 130R1. The chemical structures of such agents are well known in the art. In one embodiment, the agent has a hydrophile-lipophile balance (HLB) of between 0 and 2, as defined by Hunter and Bennett (J. Immun. 133:3167, 1984). The agent can be provided in an effective amount, for example between 0.5 and 10%, or in an amount between 1.25 and 5%. The oil included in the composition is chosen to promote the retention of the poly-ICLC in oil-in-water emulsion, and can have a melting temperature of less than 65°C, such that emulsion is formed either at room temperature, or once the temperature of the emulsion is adjusted to room temperature. Examples of such oils include squalene, squalane, EICOSANE™, tetratetracontane, glycerol, and peanut oil or other vegetable oils. In one specific, non-limiting example, the oil is provided in an amount between 1 and 10%, or between 2.5 and 5%. The oil should be both biodegradable and biocompatible so that the subject can break down the oil over time, and so that no adverse affects, such as granulomas, are evident upon use of the oil.

As an alternative to liquid formulations, the poly-ICLC-containing composition can be administered in solid form, e.g., as a powder, pellet or tablet. For example, the formulation can be administered as a powder using a transdermal needleless injection device, such as the helium powered POWDERJECT® injection device. This apparatus uses pressurized helium gas to propel a powder formulation of a poly-ICLC -containing composition at high speed so that the particles perforate the stratum corneum and contact cells in the epidermis.

Polymers can be also used for controlled release. Various degradable and nondegradable polymeric matrices for use in controlled drug delivery are known in the art (Langer, Accounts Chem. Res. 26:537, 1993). For example, the block copolymer olaxamer can exist as a viscous yet mobile liquid at low temperatures but forms a semisolid gel at body temperature (Johnston et al., Pharm. Res. 9:425, 1992; and Pec, J. Parent. Sci. Tech. 44(2):58, 1990). Alternatively, hydroxyapatite has been used as a microcarrier for controlled release (Ijntema et al., Int. J. Pharm. 112:215, 1994). In yet another aspect, liposomes are used for controlled release as well as drug targeting of the lipid-capsulated drug (Betageri et al., Liposome Drug Delivery Systems, Technomic Publishing Co., Inc., Lancaster, Pa., 1993). Numerous additional systems for controlled delivery of therapeutic compositions are known (e.g., U.S. Pat. Nos. 5,188,837; 4,235,871; 4,501,728; 4,837,028; 4,957,735; 5,019,369; 5,055,303; 5,514,670; 5,413,797; 5,268,164; 5,004,697; 4,902,505; 5,506,206; 5,271,961; 5,254,342; and 5,534,496).

***Treatment with pharmaceutical compositions that include poly-ICLC as an ingredient***

The present disclosure concerns methods of protecting cells, tissues, and organs *in vivo,* from an excitotoxic injury resulting from an ischemic or other hypoxic event by administering poly-ICLC systemically to a subject at risk of developing an excitotoxic injury.

The present disclosure also concerns compositions that include poly-ICLC as an ingredient for use in protecting cells, tissues and organs in vivo from excitotoxic injury resulting from an ischemic or other hypoxic event that are formulated to be administered systemically.

The methods disclosed herein are applicable to any cell or tissue type that is at risk of developing an excitotoxic injury due to an ischemic or hypoxic event. For example, neural cells and tissues including neural cells (including, e.g., neurons, astrocytes and glial cells in either the central or peripheral nervous system), muscle cells and tissues including muscle cells, (including cardiac, smooth and striated muscle cells), hepatic cells and tissues including hepatic cells, renal cells and tissues including renal cells, lymphocytes and tissues including lymphocytes, and endothelial cells and tissues including endothelial cells can be protected from the damage resulting from excitotoxic, ischemic, and/or hypoxic injury using the methods disclosed herein.

Excitotoxic injury results from excessive stimulation of cells (for example neural cells in the CNS) by certain neurotransmitter (e.g., glutamate) receptors. For example, excitotoxic injury can be a result of a condition that causes excessive chemical or electrical activity in the brain or it can be a result of conditions that cause a decrease in inhibitory or regulatory functions of the brain. Excitotoxic injury in the brain is associated with a variety of conditions with disparate etiologies and symptoms, including epilepsy, traumatic brain injury, Alzheimer's disease, and hypoxia, including hypoxia caused by ischemia. In some examples, subjects who have previously suffered from such conditions (whether or not they have previously suffered excitotoxic injury or not) are at risk of developing an excitotoxic injury.

Hypoxia in the central nervous system (CNS) can be associated with ischemic events (such as cerebrovascular ischemia, stroke, myocardial ischemia due to narrowing or blockage of the vessels of the heart, iatrogenic ischemia, due to surgical procedures, and the like). In addition, hypoxia can occur in utero due to conditions such as inadequate placental function (for example, due to abrupio placentae), preeclamptic toxicity, prolapse of the umbilical cord, or complications from anesthetic administration. Ischemic events outside the CNS can also result in injury to tissues and organs, including kidney, liver, lung, intestine, and muscle.

Hypoxic injury can be the result of vascular disease or injury, as well as a complication of surgical procedures (e.g., cardiovascular surgery). Additionally, injury by some hypoxic events (such as strokes) involves an excitotoxic component as well as a hypoxic component and are, in some but not all cases related to ischemic events. In one example, the subject at risk of hypoxic injury has suffered from or is at risk for: atrial fibrillation, one or more transient ischemic events, a stroke, hypertension, or combinations thereof.

For simplicity of reference, in the context of this disclosure, the term "cytotoxic insult" may be used to refer to any of these conditions, separately or in any combination. The methods disclosed herein are useful for preventing or treating cellular damage in any (and/or all) of these conditions.

Accordingly, the methods can involve selecting a subject who is at risk of developing a hypoxic injury, such as a hypoxic injury resulting from ischemia. Subjects at risk of developing a hypoxic injury include those subjects who developed one or more hypoxic injuries in the past.

Biological signs of risk of developing hypoxic injury, particularly those caused by ischemic events may be observed and/or monitored and/or measured for example, by Computed Axial Tomography (CT scan, CAT scan); Magnetic Resonance Imaging (MRI scan, MR scan); Carotid Ultrasound, including Transcranial Doppler (TCD); Cerebral Angiography: (Cerebral arteriogram, Digital subtraction angiography [DSA]); Computed Tomographic Angiography: (CT-angiography, CT-A, CTA); Magnetic Resonance Angiography (MRA) and/or other diagnostic procedures known to those of ordinary skill in the art. The results of one or more of the above diagnostic procedures can be used to select a subject for administration of poly-ICLC.

In particular embodiments, the level of hypoxia or ischemia in a cell, tissue, or subject is measured in a subject, for example a subject who has previously had, or is at risk for, an ischemic or hypoxic event. In one embodiment, the level of hypoxia or ischemia in a cell, tissue, or subject is measured in a subject in need of administration of poly-ICLC. Methods of measuring hypoxia or ischemia in a cell, tissue, or subject are well known to those of skill in the art and can include, for example, measuring the oxygen level in the cell, tissue, or subject.

In the context of the preconditioning methods described herein, risk is indicated by a variety of medical as well as non-medical indicators, as would be recognized by one of ordinary skill in the art. For example, various cardiovascular signs and symptoms, such as atrial fibrillation, angina pectoris, hypertension, transient ischemic attacks and prior stroke, are all indicators of risk that can be used to select a subject for administration of preconditioning agent according to the methods disclosed herein.

Similarly, surgical procedures, especially those specifically involving the cardiovascular system, such as endarterectomy, pulmonary bypass and coronary artery bypass surgeries, or abdominal surgeries are indicators of risk that can be used to select a subject for administration of a composition that includes poly-ICLC. Intestinal ischemic events including chronic or acute mesenteric ischemia are associated with risk factors including advanced age, patients with postprandial abdominal pain and weight loss, chronic or acute colitis, cardiac arrhythmia, recent heart attack or prior emboli, prior arterial insufficiency, diabetes, hypertension, trauma, congestive heart failure, infection or inflammation and prior use of vasoconstrictor drugs. Thus, a preconditioning agent (e.g., poly-ICLC) can be administered to a subject that has been identified as having (e.g., diagnosed with) one or more risk factors indicative of an increased likelihood, relative to the general population or to a subject without the risk factor, of having an excitotoxic, ischemic and/or hypoxic event.

In addition, non-medical indicators of risk, for example, pertaining to behaviors or activities that are statistically associated with an increased likelihood of injuries, can include an excitotoxic or hypoxic component. For example, traumatic brain injury (regardless of its cause) frequently involves a hypoxic component. Thus, participation in activities that increase the risk of traumatic brain injury is an indicator that can be used to select a subject for administration of composition that includes poly-ICLC. Such activities include, for example, motorcycle riding, motor vehicle racing, skiing, contact/combat sports (such as, football, hockey, rugby, soccer, lacrosse, martial arts, boxing and wrestling), and the like. Additionally, impacts or wounds resulting from gunshot or explosives frequently cause traumatic brain injury. Accordingly, activities that are associated with an increased risk of gunshot wounds or injury caused by explosive devices (for example, in combat or military situations) are indicators of risk that can be used to select a subject for treatment according to the methods disclosed herein.

Commonly, the composition containing the poly-ICLC is a pharmaceutical composition or medicament, formulated for administration to a subject, including such a composition formulated to be administered systemically to the subject. Such compositions commonly include a pharmaceutical carrier or excipient. Generally, the composition is formulated based on the intended route of administration. Suitable routes of administration include intranasal, oral, transdermal, subcutaneous, intrathecal, intravenous, intramuscular, and intraperitoneal routes, and appropriate pharmaceutical carriers for these administration routes are well known in the art. Thus, the use of a composition that includes poly-ICLC in the preparation of a medicament for the treatment or prevention of a hypoxic injury is a feature of this disclosure.

One particular example of a composition that includes poly-ICLC is poly-ICLC alone or poly-ICLC suspended in a buffered or other solution without any additional additives. In another particular example, the carrier may be a liposome or any other molecular structure that can protect a pharmaceutical composition including poly-ICLC from degradation in the serum (See Wong, US Patent Number 6,468,558, filed 19 Oct 2001). Alternatively, the pharmaceutically acceptable carrier may include purified poly-ICLC without any additional fluid or other carrier.

In one example, the composition that includes poly-ICLC is administered after an event that increases the risk of hypoxic injury in order to protect against any hypoxic injury resulting from the event. In some examples, the composition including poly-ICLC is administered with other therapeutic agents, such as tissue plasminogen activator (tPA). In one example, the composition that includes poly-ICLC is administered in at least one dose at least 1 hour after the event, such as at least 2, 4, 6, 12, 24,48 or 72 hours after the event, for example at least 7 days, at least 10 days, or at least 30 days after the event, but prior to the development of any ischemia or hypoxic injury resulting from that ischemia.

Optionally, multiple doses of the poly-ICLC-containing composition are administered after the event. For example, two, or three, or more doses can be administered on separate occasions following the event. In some embodiments, a first dose is given between 1 and 72 hours, at seven days, at six days, at five days, at four days, at three days, at two days, or at 1 day following the event. One or more subsequent administrations of the compositions can be made at any subsequent time point, such as at seven days, at six days, at five days, at four days, at three days, at two days, at 24 hours or at 12 hours following the event. In some examples, multiple administrations are given following the event (such as for weeks, months or years following the event), but prior to the onset of the hypoxic injury.

For example, in the case of an ongoing event or series of events, multiple administrations are given, for example on a predetermined schedule, such as at weekly intervals. Alternatively, the composition can be formulated and administered on a continuous basis, for example using a pump (or other intravenous or intrathecal) infusion method. The individual treatment regimen can be customized to the particular event or activity, such that the therapeutic effects of the dose of the poly-ICLC are optimized under the particular circumstances for the particular subject.

In another example, the composition that includes poly-ICLC is administered prior to an event or activity that increases the risk of excitotoxic injury, ischemia and/or hypoxia, in order to protect the cells from adverse consequences resulting from such events. For example, at least one dose of poly-ICLC-containing composition can be administered at least 10 hours prior to the event or activity, in order to better realize the preconditioning effect of administration.

Usually, the composition is administered at least 24 hours before the event or activity. The protective effects of a single administration of a compound including poly-ICLC can last for longer than one week (e.g., up to about 10 days, or more). Thus, in the case of an isolated event, that is, an event that is not predicted to be a recurring event (such as a surgical procedure), the composition may be administered prior to the commencement of the event, such as about 10 hours, or about 12 hours, or about 24 hours prior to the event or activity, and can be administered up to about 1 week prior to the event. Optionally, multiple doses of the composition are administered prior to the commencement of the event (e.g., surgery). For example, two, or three, or more doses can be administered on separate occasions preceding the event. In some embodiments, a first dose is administered between 8 and 10 days, at seven days, at six days, at five days, at four days, at three days, at two days, or at 1 day prior to the event. One or more subsequent administrations of the compositions can be made at any subsequent time point, such at seven days, at six days, at five days, at four days, at three days, at two days, at 24 hours or at 12 hours prior to the event.

In the case of a recurrent event, such as repeated engagement in a contact sport, multiple doses are administered, with the dose administered nearest in time to the event administered prior to (such as, at least 10 hours, or up to about 1 week, prior) to the event or activity. Other doses are administered prior to the dose administered nearest in time to the event. Similarly, in the case of an ongoing event, such as in the case of Alzheimer's disease, multiple doses are administered, for example on a predetermined schedule, such as at weekly intervals. The individual treatment regimen can be customized to the particular subject event or activity, such that the treatment or protective effects of the composition including poly-ICLC are optimized under the particular circumstances for the particular subject.

Exemplary doses of poly-ICLC that can be used in the methods provided herein {for example, in a human) include at least 0.005 mg/kg, such as at least 0.05 mg/kg, at least 0.01 mg/kg, at least 0.1 mg/kg, at least 1 mg/kg, or at least 2 mg/kg of the poly-ICLC containing composition. In one example, the dose contains no more than about 0.2 mg/kg, no more than about 0.5 mg/kg, no more than about 1 mg/kg, or no more than about 2 mg/kg of the poly-ICLC containing composition. For example, a dose can include between 0.01 mg/kg and 10.0 mg/kg, 0.005 mg/kg to 5 mg/kg, 0.1 mg/kg to 4 mg/kg of a poly IC-containing composition, such as between 1.0 mg/kg and 2 mg/kg. Certain exemplary doses include about 0.07, about 0.08, about 0.09, about 0.10, about 0.12, about 0.15, about 0.9, about 1, about 1.6, about 2, about 5, or about 10 or more mg/kg.

Precise doses of the composition will depend upon the stimulatory capacity of the individual ligand formulation and human in vivo absorption, distribution, metabolism and ultimately the bioavailability of the composition. In light of this disclosure, these precise doses are all available to those of skill in the art with reasonable experimentation.

## EXAMPLES

### *Example 1- Materials and Methods*

The following example describes the materials used and experimental methods followed in Examples 2-4 below.

<u>Mice:</u> Animal procedures were conducted using C57/Bl6 male mice, 8- to 12-week old (Jackson Laboratory, West Sacramento, CA, USA), according to Oregon Health and Science University (OWLAW #A3304-01) Institutional Animal Care and Use Committee and National Institute of Health Guidelines. The American Association for Laboratory Animal Care accredits the housing facility.

<u>Compositions:</u> Carboxymethylcellulose (Sigma-Aldrich, St Louis, MO, USA), poly-ICLC (Hiltonol; Oncovir, Washington, DC, USA), saline, poly-IC high molecular weight (Invivogen, San Diego, CA, USA), and LPS (Escherichia coli serotype 0111:B4; phenol extraction purified, protein content _3%; Sigma-Aldrich) were used. Agents were delivered by either intraperitoneal or subcutaneous administration as noted in Examples 2- 4 below.

<u>Oxygen Glucose Deprivation Procedure:</u> Primary mixed cortical cultures were prepared from E15 to E17 mouse fetuses (1 litter/experiment) as previously published (Stevens SL et al, J Cereb Blood Flow Metab 28, 1040-1047 (2008), incorporated by reference herein. During oxygen glucose deprivation (OGD), medium was replaced with D-PBS (Gibco, Carlsbad, CA, USA) and cells were incubated in an anaerobic atmosphere of 85% $N_2$, 10% $CO_2$, 5%$H_2$ at 37°C for 3 hours (Coy Laboratories, Grass Lake, MI, USA). After oxygen glucose deprivation, D-PBS was replaced with medium and cells returned to a normoxic incubator. Control plates remained in the normoxic incubator during oxygen glucose deprivation. Cell death was determined 24 hours after oxygen glucose deprivation using propidium iodide staining (Sigma-Aldrich) and quantified with Metmorph7® software (Molecular Devices, Downington, PA, USA). Within an experiment, each treatment was performed in triplicate and three independent experiments were performed.

<u>Middle Cerebral Artery Occlusion Procedure:</u> Mice were treated with poly-ICLC (0.4, 0.8 or 1.6 mg/kg), LPS (1 mg/kg), or appropriate vehicle (subcutaneous; n=9 to 10/group) 72 hours before middle cerebral artery occlusion (MCAO) (45 minutes) performed as previously described (Stevens et al, 2008, *supra).* Laser Doppler (Transonic Systems Incorporated, Ithaca, NY, USA) was used to measure cerebral blood flow through the skull adjacent the middle cerebral artery to insure occlusion reduced flow to 20% of baseline. About 24 hours after MCAO, neurologic testing was administered with experimental subjects blinded to the experimenter performing the testing. In addition, indirect infarct volume determined by 2,3,5 triphenyltetrazolium chloride stain. There are two categories of neurologic scoring each based on a 28-point scale which correlates with infarct volume (Clark WM et al, Neurol Res 19, 641-648 (1997), incorporated by reference herein.)

The general neurological score evaluates activity, posture, and grooming. The focal neurological score evaluates body symmetry, gait, and traits characteristic of unilateral MCAO damage. The corner test evaluates body function and asymmetry by noting direction turned when mice approach a 45° corner. After middle cerebral artery occlusion, mice tend to favor their ipsilateral (right) side turning preferentially right when exiting while untreated mice turn equally to either side. Each mouse was scored 10 consecutive times to calculate the percentage of right hand turns (Zhang L et al, J Neurosci Methods 117, 207-214 (2002), incorporated by reference herein.

Renal Ischemia Procedure: Mice were administered vehicle, LPS (1.6 mg/kg), or poly-IC (intraperitoneal; 1.6 mg/kg) 48 hours before surgery (n = 6/group). Renal ischemia was induced in isoflurane (1.5% to 2%) anesthetized mice by performing a midline incision, isolating the renal vessels bilaterally, clamping the renal vessels with small bulldog clamps (45 minutes), and confirming occlusion and reperfusion by Parks Ultrasonic 811-B Doppler (Kroslak Enterprises, Riverview, FL, USA). Blood samples were taken via saphenous vein before renal ischemia surgery and 48 hours after reperfusion for creatinine level determination by the Jaffe rate method using a Beckman Coulter DXC800 (Brea, CA, USA).

Plasma Cytokine Evaluation: Blood was collected via cardiac puncture under isoflurane anesthesia 3 hours after subcutaneous administration of vehicle, LPS (1 mg/kg), or poly-ICLC (1.6 mg/kg) (n=3/group). Plasma cytokine levels were evaluated by custom multiplex ELISA for IL-1β, IL-12, IFNγ (Quansys Biosciences, Logan, UT, USA) and cytometric bead array for IL-6 and TNFα (BD Biosciences, Franklin Lakes, NJ, USA).

Data Analysis: Researchers were blinded to treatment during analyses. Significance (P < 0.05) was determined using Student's t-test or one-way analysis of variance (ANOVA) with Bonferroni's post hoc test as noted using GraphPad Prism 5® software (GraphPad, La Jolla, CA, USA).

### Example 2 - Poly-ICLC Preconditioning Confers Neuroprotection in vitro

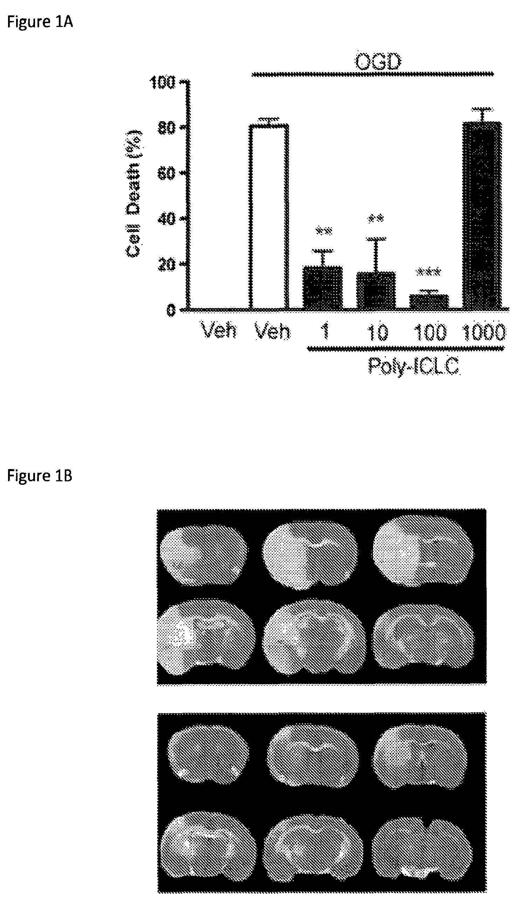

Mixed cortical cultures were used to test whether poly-IC preconditioning provides protection against modeled ischemia in vitro. Preconditioning with poly-ICLC (1 to 100 ng/ml) produced a marked attenuation of cell death after oxygen glucose deprivation compared with controls. Cells pretreated with poly-ICLC showed little cell death after oxygen glucose deprivation with the greatest protection exhibited at 100 ng/ml with 5.6 ± 2.4% cell death compared with 80.6 ± 3.1% cell death in vehicle-treated controls (Figure 1A). Cell death was the same as that of vehicle at 1000 ng/ml.

### Example 3 - Poly-ICLC Preconditioning Confers Neuroprotection in vivo

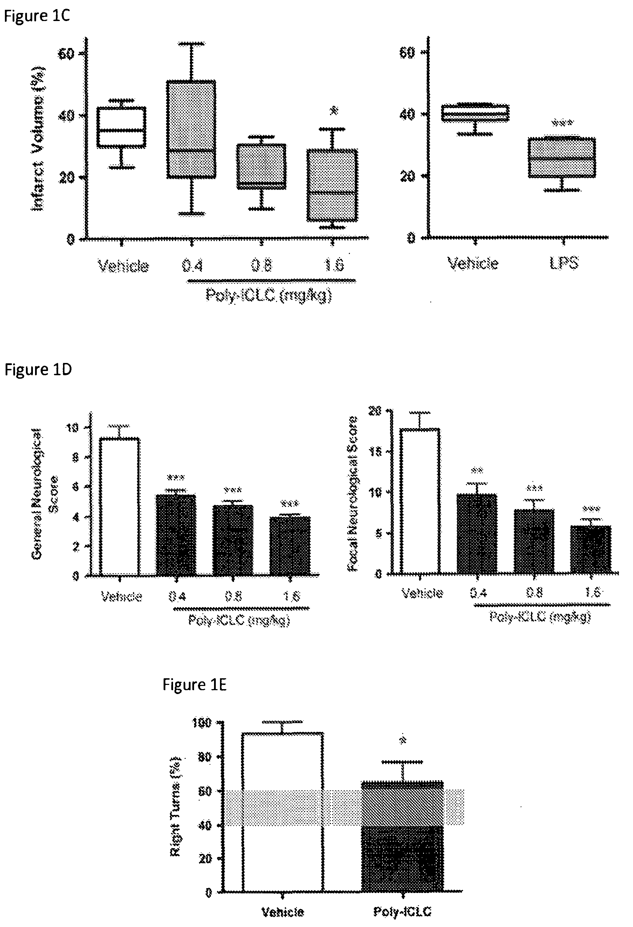

Poly-ICLC preconditioning was then used to protect the brain from ischemic injury in an *in-vivo* mouse model of stroke. Poly-ICLC (0.4, 0.8, or 1.6 mg/kg) was administered 3 days before MCAO as this time point has shown consistent protection with other preconditioning stimuli including LPS. The infarct volume determined 24 hours after middle cerebral artery occlusion was significantly smaller in poly-ICLC-treated animals - animals treated with 1.6 mg/kg poly-ICLC had a 17.64 ± 3.89% infarct volume, while vehicle treated controls had a 35.29 ± 2.41% infarct volume with a *P* < 0.05. Results are depicted in Figures 1B and 1C. The degree of protection was similar to the known preconditioning agent, LPS (Figure 1C).

As with other preconditioning stimuli, the lower infarct volume upon treatment was dose-dependent with higher doses of poly-ICLC resulting in a smaller infarct volume resulting from middle cerebral artery occlusion. Poly-IC administered at a higher dose (4 mg/kg) neither conferred neuroprotection nor exacerbated damage (vehicle 41.58 ± 3.06%, poly-IC 43.72 ± 2.96%; P > 0.05).

Neurologic and motor deficits were attenuated in poly-ICLC preconditioned mice as evidenced by lower general and focal neurologic scores for poly-ICLC treated mice relative to controls (Figure 1D) and improved performance in the corner test, in which animals preconditioned with poly ICLC showed a decreased tendency to turn to the ipsilateral side compared with vehicle-treated controls (Figure 1E).

### Example 4 - Poly-ICLC Treatment Elicits Lower Systemic Plasma Cytokine Levels Relative to Lipopolysaccharide

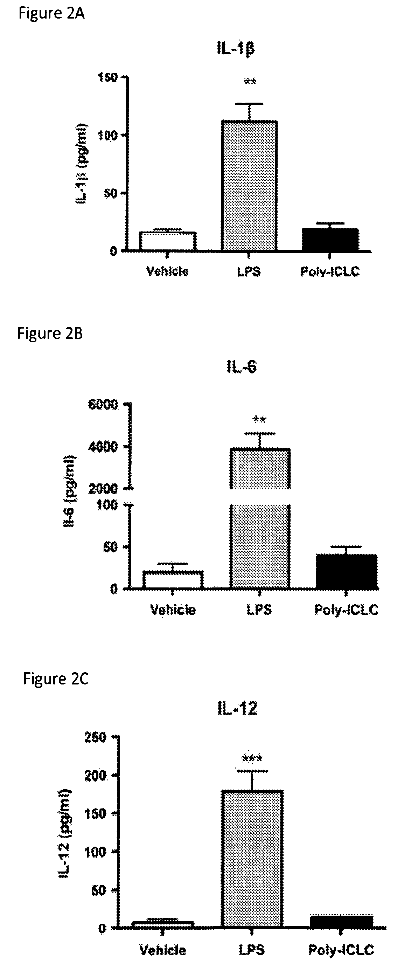

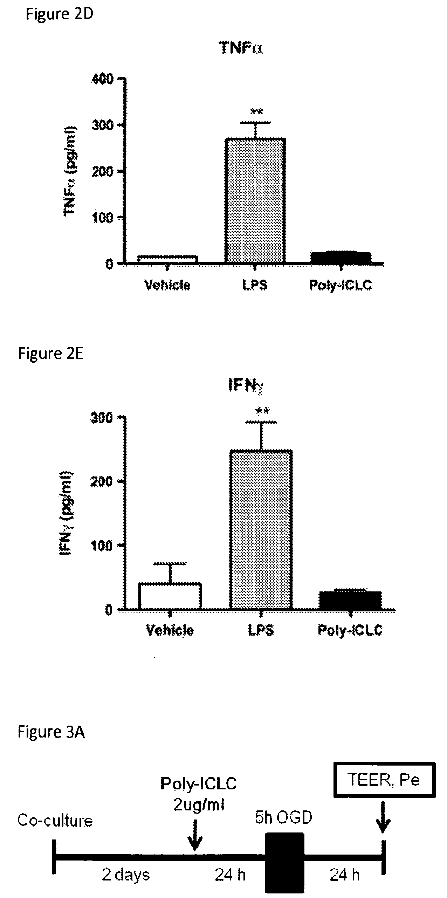

While LPS preconditioning induces ischemic tolerance in multiple organs, the pro-inflammatory properties of LPS limit its use in a therapeutic setting. Poly-ICLC on the other hand, elicits a much lower cytokine response than LPS. Plasma cytokine concentrations were assessed 3 hours after administration of poly-ICLC or LPS known to be protective against ischemic damage in Examples 2-3 above. Mice treated with poly-ICLC had significantly lower plasma concentrations of IL-1β, IL-6, IL-12, TNFα and IFNγ compared with mice treated with LPS (P < 0.01; Figures 2A-2E).

*Example 5 - Materials and Methods II*

The following example describes the materials used and experimental methods followed in Examples 6-11 below.

Mice: C57Bl/6J (WT) mice were obtained from Jackson Laboratories (West Sacramento, CA). Type I IFN receptor deficient (IFNAR-/-) mice were provided by Dr. Herbert Virgin and Dr. Anthony French (Washington University School of Medicine, St. Louis, MO). IFNβ-/- mice were provided by Dr. Tomas Leanderson (Lund University). All in vivo studies were performed with male mice between 8-12 weeks of age. Primary cultures were prepared from 1-2 day postnatal mice (mixed glial) or 8-12 week-old male mice (endothelial). All mice were given free access to food and water and housed in a facility approved by the Association for Assessment and Accreditation of Laboratory Animal Care International. Animal protocols were approved by the Oregon Health & Science University Institutional Animal Care and Use Committee (OWLAW# A3304-01) and met the guidelines set forth by the National Institutes of Health.

Drug treatments: For in vivo experiments mice were given a subcutaneous injection of poly-ICLC (also known as Hiltonol®), which is poly-IC stabilized with poly-L-lysine and carboxymethyl cellulose; 1.6 mg/kg, Oncovir, Washington DC) or carboxymethyl cellulose vehicle (Sigma Aldrich, St. Louis MO) in a total volume of 100 μl. Mice were treated 72 hours prior to the induction of ischemia. For in vitro experiments both compartments of the co-cultures were treated with vehicle or poly-ICLC (2μg/ml) 24 hours prior to OGD.

Blood-Brain-Barrier in vitro model: Primary cultures of mixed glial cells, containing both astrocytes and microglia, were prepared from 1-2 day postnatal mice (Kis B et al, Neuroreport 12, 4139-4142 (2001), incorporated by reference herein). Brains were collected, meninges removed and cortical pieces were mechanically dissociated and incubated for 15 minutes at 37°C with trypsin (2.5mg/ml; Invitrogen, CA, USA) and DNAse I (0.08mg/ml; Sigma, MO, USA). Supernatant containing the astroglial cells was collected, centrifuged and resuspended in Dulbecco's modified Eagle medium (DMEM, Invitrogen) containing 0.5mg/ml gentamicin (Sigma) and 10% fetal bovine serum (FBS, HyClone, MA, USA). Cells were seeded on poly-L-lysine (Sigma) coated 12-well plates. Glial cells were cultured for 3 weeks before use in experiments. Brain microvessel endothelial cells (BMECs) were prepared from male mice (8-12 week-old) (Deli MA et al, Cell Mole Neurobiol 25, 59-127 (2003), incorporated by reference herein). Forebrains were collected in ice-cold sterile phosphate buffered saline (PBS) and the meninges were removed. Subsequently, the gray matter was minced to 1 mm$^3$ pieces and digested with 1 mg/ml collagenase CLS2 (Worthington, NJ, USA) in DMEM for 45 minutes at 37 °C. Microvessels were separated from myelin containing elements by centrifugation in 20% bovine serum albumin (Sigma)-DMEM (1000 x g, 20minutes), and further digested with 1 mg/ml collagenase-dispase (Roche, Mannheim, Germany) in DMEM for 30 minutes to remove pericytes from the basal membrane. Microvascular endothelial cell clusters were separated on a 33% continuous Percoll (Amersham, NJ, USA) gradient, collected, and seeded on collagen type IV and fibronectin coated cell culture inserts (Transwell clear, 1.12 cm2; pore size, 0.4 μm, Costar, MA, USA). Cultures were maintained in DMEM supplemented with 5 μg/ml gentamicin (Sigma), 20% plasma-derived bovine serum (First Link, UK), 200 μg/ml endothelial cell growth supplement (Sigma) and 100 μg/ml heparin (Sigma). In the first 2 days, culture medium contained 4 μg/ml puromycin (Sigma) to selectively remove P-glycoprotein negative contaminating cells (Perriere N et al, J Neurochem 93, 279-289 (2005), incorporated by reference herein). Cultures reached confluency within 4-5 days and then were used for experiments.

To induce typical BBB characteristics, BMECs were co-cultured with mouse cerebral astrocytes and microglia cells (Deli et al, Inflamm Res 52 Suppl1 S39-S40 (2003); incorporated by reference herein and Deli et al, 2005 *supra*). Two days after seeding, the transwell inserts containing the BMECs were placed into multiwell plates containing the glial cells at the bottom and the culture medium was replaced with fresh endothelial culture medium. Twenty-four hours later, culture medium was supplemented with 8-(4 Chlorophenylthio)adenosine 3',5'-cyclic monophosphate sodium salt (CPT-cAMP) 250 μM (Sigma, MO, USA) and 4-(3-Butoxy-4-methoxybenzyl)imidazolidin-2-one (RO 201724) 17.5 μM (Sigma, MO, USA) for 24 hours to tighten junctions and elevate resistance (Rubin LL et al, J Cell Biol 115, 1725-1735 (1991), incorporated by reference herein; Deli et al 2005 *supra* and Perriere et al 2005 *supra*).

Measurement of monolayer resistance and permeability: Trans-endothelial electrical resistance (TEER), representing the permeability of tight junctions for sodium ions, was measured by an EVOM resistance meter (World Precision Instruments, FL, USA) using STX-2 electrodes and was expressed relative to the surface area of endothelial monolayer (Ωcm$^2$). The trans-endothelial electrical resistance of cell-free inserts (80-90 Ωm$^2$) was subtracted from the values. For permeability measurements the flux of Na-fluorescein (Sigma) was used as an index of paracellular transport, and peroxidase-conjugated albumin (Jackson ImmunoResearch, PA, USA) was used as an index of transcelluar pathway across endothelial monolayers. These values were determined as previously described in Kis et al, 2001 supra.

Briefly, cell culture inserts with BMECs were transferred to 12-well plates containing 1.5 ml/well of DMEM without phenol red. In upper chambers culture medium was replaced by 500 μl DMEM without phenol red containing 10 μg/ml Na-fluorescein (MW: 376 Da) and 0.5 μg/ml of peroxide albumin (MW: 67 kDa). After 15, 30, 60, 120 and 240 minutes the inserts were transferred to a new well containing DMEM without phenol red. The concentrations of the marker molecules were determined in samples obtained from the lower compartments. Albumin and Na-fluorescein concentrations were measured by a plate reader (Spectra Max 190, absorbency 450nm for Albumin and Spectra Max Gemini XS emission: 515 nm, excitation: 460 nm for Na-fluorescein, both from Molecular Devices, CA, USA). Flux across cell-free inserts was also measured. Transport was expressed as '$\mu$l' of donor (upper) compartment volume from which the tracer is completely cleared (Deli et al. 2005, supra):

$$\text{cleared volume } (\mu l) = \text{concentration}_{lower} \times \text{volume}_{lower} \times \text{concentration}^{-1}_{upper}$$

The average cleared volume was plotted versus time, and permeability x surface area product value for endothelial monolayer (PSe) was calculated by the following formula:

$$PS^{-1}_{endothelial} = PS^{-1}_{total} - PS^{-1}_{insert}$$

PSe divided by the surface area generated the endothelial permeability coefficient (Pe, in $10^{-3}$ cm/min).

Oxygen-glucose deprivation (OGD): Co-cultures were incubated in an anaerobic atmosphere of 85% $N_2$, 10% $CO_2$, 5% $H_2$ at 37°C for 5 hours. Once in the hypoxic chamber, the culture medium was replaced with deoxygenated glucose-free medium. The anaerobic conditions within the chamber were monitored using an electronic oxygen/hydrogen analyzer (Coy Laboratories, Grass Lake MI). After the oxygen-glucose deprivation period, medium was replaced with normoglycemic medium and the cells were returned to a normoxic incubator (reoxygenation) for up to 24 hours. In control co-cultures the medium was replaced with fresh complete culture medium and the cells were not exposed to oxygen-glucose deprivation.

Immunofluorescence: BMECs were fixed with ice-cold ethanol for 30 minutes at 4°C. After 30 minutes incubation with 0.1% Triton® for permeabilization followed by 30 minutes with 3% fetal bovine serum for blocking, the cells were incubated with the primary antibodies rabbit anti-ZO-1 (1:100; Zymed, CA, USA) and rabbit anti-occludin (1:100; Zymed, CA, USA) overnight at 4°C. Anti-rabbit secondary antibody (1:100, Chemicon) was used. Immunofluorescent staining was acquired by an Olympus BX61® microscope equipped with an Olympus confocal scan unit FV1000®.

IFN$\alpha$ and IFN$\beta$ ELISA: Culture media were collected at 6, 8 and 24 hours after poly-ICLC treatment and 24 hours after oxygen glucose deprivation. The media was immediately concentrated using centrifugal filters (Amicon Ultra, 3K, Millipore, MA, USA) for 30 minutes at 14,000g and then stored at -80°C until analysis. The IFN$\alpha$ and IFN$\beta$ levels in the culture media were measured by ELISA kits (PBL Interferon Source, NJ, USA) according to manufacturer's instructions.

RNA isolation, reverse transcription, and quantitative PCR: RNA was isolated from both endothelial and glial cells from individual wells at 6, 8 and 24 hours after poly-ICLC treatment and 24 hours after OGD using a Mini® RNA isolation kit (Qiagen, CA, USA). Reverse transcription was performed on 1 $\mu$g of RNA using Omniscript® (Qiagen). Quantitative PCR was performed using Taqman® Gene Expression Assays (Applied Biosystems, CA, USA) for each gene of interest on StepOne Plus® machine (Applied Biosystems). Results were normalized to $\beta$-Actin expression and analyzed relative to their control counterparts. The relative quantification of the gene of interest was determined using the comparative CT method $\Delta(2^{-\Delta\Delta Ct})$.

Mouse Ischemia-Reperfusion model: Focal cerebral ischemia was induced by middle cerebral artery occlusion (MCAO) as described previously (Stevens et al 2008, supra). In brief, MCAO was performed in anesthetized mice (1.5-2% isoflurane) by threading a 7-0 silicon-coated nylon surgical filament (Doccol, Redlands CA) through the external carotid artery to the internal carotid artery, blocking blood flow at the bifurcation of the MCA and anterior cerebral artery for 45 minutes. Following occlusion, the filament was removed and blood flow was restored. Cerebral blood flow was monitored throughout the procedure by laser Doppler flowmetry (Transonic System Inc., Ithaca NY) and animals were excluded if blood flow was not reduced by 80% or greater during occlusion. Body temperature was maintained at 37°C during the surgery.

Evaluation of infarct size: Twenty-four hours following middle cerebral artery occlusion, mice were deeply anesthetized with isoflurane and then perfused with ice-cold saline containing 2 U/ml sodium heparin. Brains with olfactory bulbs removed were sectioned into 1 mm slices beginning from the rostral end, for a total of 7 slices. The infarct area was visualized by incubating the sections in 1.5% 2, 3, 5-triphenyltetrazolium chloride (TTC, Sigma) in PBS for 10 minutes at 37°C (Sigma Aldrich). Sections were then imaged and the infarct area was measured using ImageJ software (NIH Image, Bethesda MD). Infarct volume was calculated using the indirect method [(contralateral$_{live}$ - ipsilateral$_{live}$) / contralateral$_{live}$ * 100] to account for the effects of edema, and the final infarct data are given as % damage.

Statistical analysis: Data are represented as mean $\pm$ SEM. All in vitro experiments were repeated at least two or three times with 4 wells for each time point. Figures denote combined experiments. Statistical analysis was performed using GraphPad Prism5® software. Two-way ANOVA with Bonferroni post-hoc test was used for TEER and in vivo studies, one-way ANOVA with Bonferroni post-hoc test for Pe, ELISA and mRNA analysis.

### *Example 6 - Poly ICLC preconditioning significantly attenuates oxygen-glucose deprivation-induced effects on the blood-brain barrier*

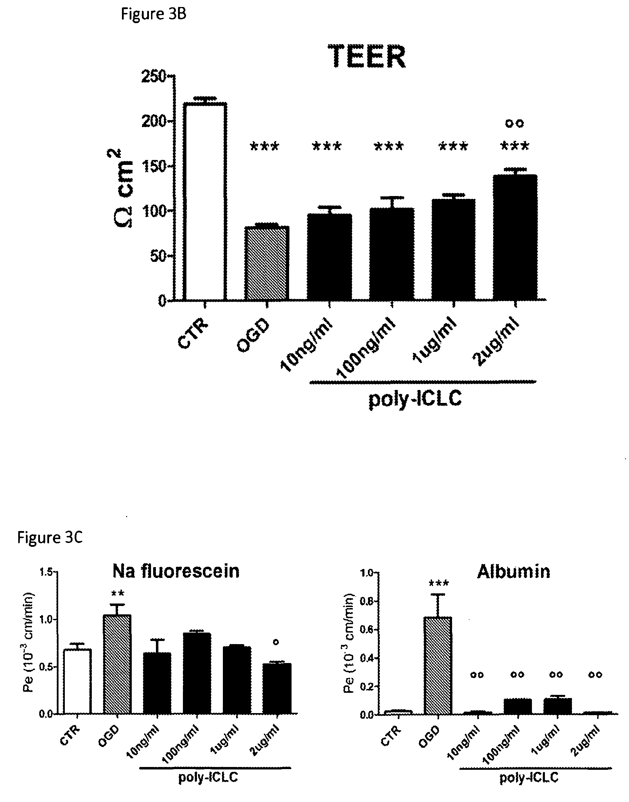

The in vitro blood-brain barrier model used for the experiments consisted of brain microvessel endothelial cells co-cultured with primary mixed astrocytes and microglia cells. Here we evaluated the effect of poly-ICLC preconditioning on the in vitro blood brain barrier subjected to oxygen glucose deprivation (Fig.3A). Poly-ICLC (10ng/ml to 2$\mu$g/ml) was administered 24 hours prior to 5 hours oxygen-glucose deprivation. The trans-endothelial electrical resistance (TEER) and endothelial permeability coefficient (Pe) for Na fluorescein and Albumin of brain microvessel endothelial cells were evaluated at 24 hours post oxygen glucose deprivation (Fig.3A). After four days of co-culture, control brain microvessel endothelial cells showed high trans-endothelial electrical resistance (218.8 $\pm$ 6.077 $\Omega$m$^2$) and low Pe values for Na fluorescein (0.67 $\pm$ 0.062 x 10$^{-3}$ cm/min) and Albumin (0.022 $\pm$ 0.0049 x 10$^{-3}$ cm/min) (CTR, Fig. 3B and 3C). These values are typical for the murine blood brain barrier model and considered representative of a functional BBB (Deli et al. 2005 supra and Gesuete et al, Stroke 42, 1445-1453 (2011); incorporated by reference herein.)

Subjecting the cells to oxygen-glucose deprivation resulted in a significantly lower trans-endothilal electrical resistance value (81.39 $\pm$ 3.5 $\Omega$m$^2$) and a significantly higher endothelial permeability coefficient (Pe) for both Na fluorescein (1.04 $\pm$ 0.12 x 10$^{-3}$cm/min) and Albumin (0.68 $\pm$ 0.16 x 10$^{-3}$cm/min) than to control co-cultures at 24 hours after oxygen-glucose deprivation. Co-cultures pretreated with 2 $\mu$g/ml poly-ICLC showed trans-endothilal electrical resistance values significantly higher (137.9 $\pm$ 7.7 $\Omega$m$^2$, Fig.3B) and Pe values for Na fluorescein significantly lower (0.52 $\pm$ 0.026 x10$^{-3}$cm/min, Fig 3C) compared to the control group subjected to oxygen glucose deprivation but pretreated with vehicle. All poly-ICLC doses tested had significantly lower Pe values for albumin compared to the control group treated with vehicle (Fig 3C). Poly-ICLC at 2$\mu$g/ml showed the best protective effect on all the outcomes measured. This dose has been used for all the following experiments.

### *Example 7 - Poly-ICLC treatment results in induction of IFN$\beta$, not of IFN$\alpha$.*

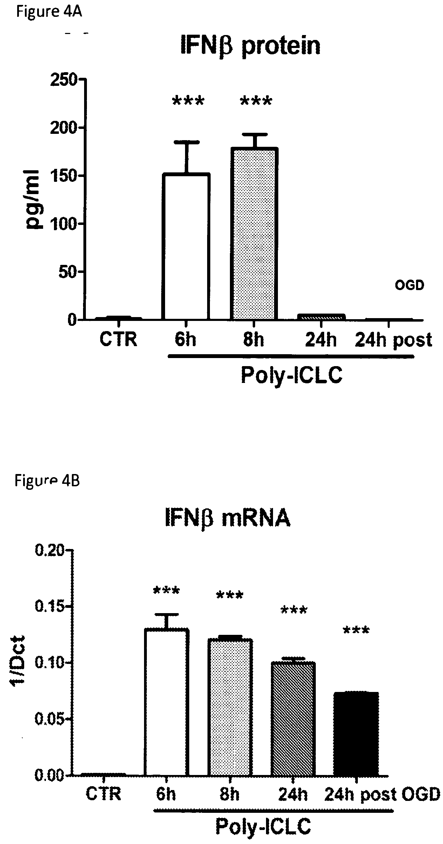

Poly-ICLC treatment is associated with robust production of both IFN$\alpha$ and IFN$\beta$ (Levy HB et al, J Infect Dis 434-439 (1975); Levy HB et al, Tex Rep Biol Med 41, 653-662 (1981); Bever CT et al, J Interferon Res 5, 423-428 (1985); Bever CT et al J Interferon Res 8,419-425 (1988); and Longhi et al, J Exp Med 206, 1589-1602 (2009); all of which are incorporated by reference herein.) To evaluate the effect of poly-ICLC on type I IFN induction in the in vitro blood brain barrier system, both IFN$\alpha$ and IFN$\beta$ were assessed in cell culture media collected at 6, 8 and 24 hours after poly-ICLC treatment and 24 hours after oxygen-glucose deprivation. Poly-ICLC treated cells had significantly more IFN$\beta$ protein at 6 and 8 hours after the treatment (151 $\pm$ 33.5 pg/ml and 178 $\pm$ 14.73 pg/ml, respectively) than control cells (1.01 $\pm$ 1.25 pg/ml, Fig. 4A). At 24 hours after poly-ICLC treatment or 24 hours after oxygen-glucose deprivation, no difference in IFN$\beta$ concentration was observed between poly-ICLC treated cells and controls. IFN$\alpha$ was not detected at any time point following poly-ICLC treatment.

IFN$\beta$ mRNA expression in both endothelial and glial cells was assessed in order to identify the cellular population producing IFN$\beta$ in the in vitro blood-brain barrier system. IFN$\beta$ mRNA was measured at 6, 8, and 24 hours after poly-ICLC treatment and 24 hours after oxygen-glucose deprivation (Fig 4B). At all the time points tested, IFN$\beta$ mRNA was expressed by glial cells treated with poly-ICLC compared to control cells (Fig 4B). Endothelial cells exhibited no IFN$\beta$ mRNA expression.

### Example 8 - IFN-$\beta$ produced by astrocytes and microglia cells mediates blood-brain barrier protection

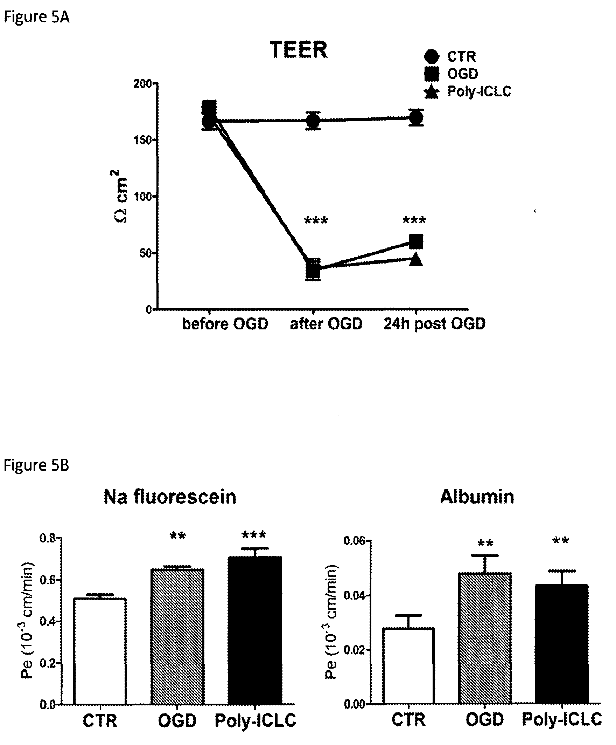

Wild type brain microvessel endothelial cells were cocultured with glial cells isolated from IFN$\beta$-/- mice in the blood-brain barrier system described above. We found that poly-ICLC preconditioning did not protect the blood-brain barrier against oxygen-glucose deprivation glial cells are unable to express IFN$\beta$. As shown in Figure 5, both trans-endothelial electrical resistance (Fig 5A) and the permeability coefficient values for Na fluorescein and albumin in the poly-ICLC treated group (Fig 5B) were similar to the vehicle-treated OGD group.

### *Example 9 - Type I IFN signaling on endothelial cells was required for poly-ICLC-induced protection*

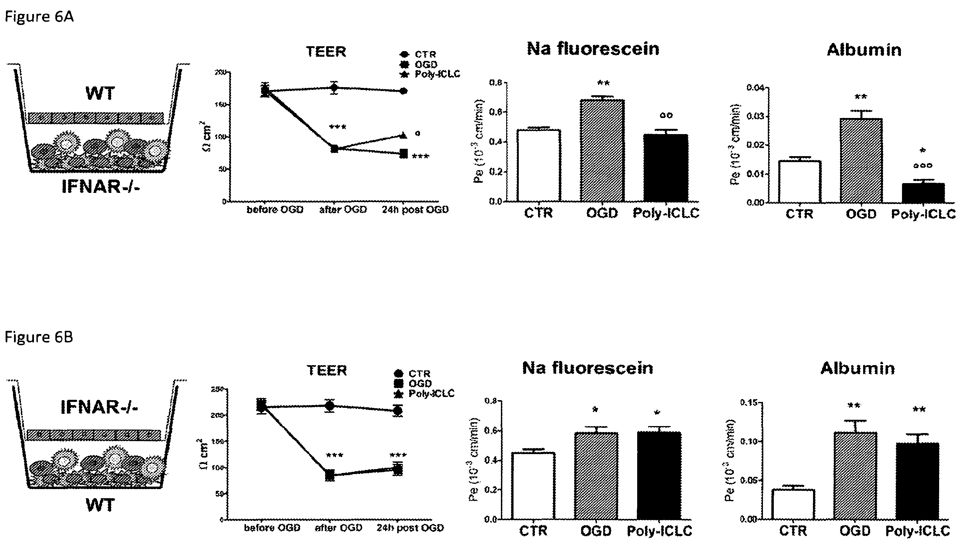

In this example, the following in vitro blood-brain barrier systems were established: wild type brain microvessel endothelial cells in co-culture with type I IFN receptor deficient (IFNAR-/-) mixed glial cells (Fig. 6A) or IFNAR-/- brain microvessel endothelial cells in co-culture with wild type mixed glial cells (Fig. 6B). Poly-ICLC preconditioning was still effective when type I IFN signaling was absent on astrocytes and microglial cells (Fig. 6A), as shown by the significant attenuation of trans-endothelial electrical resistance values (24 hours post OGD: 102.5 $\pm$ 3.9 $\Omega$m$^2$) and the permeability

coefficients for Na fluorescein ($0.45 \pm 0.033$ x$10^{-3}$ cm/min) and Albumin ($0.006 \pm 0.001$ x$10^{-3}$ cm/min) in the poly-ICLC treated group compared to oxygen-glucose deprived group (TEER 24 hours post OGD: $73.8 \pm 5.9$ $\Omega$m$^2$; Pe for Na fluorescein: $0.7 \pm 0.025$x$10^{-3}$ cm/min; Pe for Albumin: $0.03 \pm 0.002$x$10^{-3}$ cm/min). Alternatively, when type I IFN signaling was absent on endothelial cells, poly-ICLC preconditioning was no longer effective in inducing protection (Fig. 6B), indicating that type I IFN signaling on endothelial cells is necessary for poly-ICLC preconditioning.

### Example 10 - Poly-ICLC Attenuated Oxygen-glucose Deprivation-induced Tight Junction (TJ) Loss in Wild Type, but not in IFNAR-/- brain microvessel endothelial cells

Tight junction proteins play an essential role in maintaining the tightness of cell-cell interactions in blood-brain barrier endothelial cells. The presence and distribution of Zonula Occludens 1 (ZO-1), a protein located in the cytoplasm and attached to the tail of transmembrane TJ proteins and of occludin, an integral membrane TJ protein was assessed by immunofluorescence in both WT and IFNAR-/- BMECs in the preconditioning model described above. Both WT and IFNAR-/- control BMECs showed an intense and continuous staining of ZO-1 and occludin at the cell borders (Figure 7 and Figure 8, left panels). Exposure to five hours of OGD resulted in a decreased intensity, loss of the continuous junctional staining pattern, and a global disorganization of the both ZO-1 and occludin protein (Figure 7 and Figure 8, middle panels). In addition, staining intensity and distribution of ZO-1 and occudin were maintained in WT BMECs pretreated with poly-ICLC while IFNAR-/- BMECs pretreated with poly-ICLC showed a staining pattern similar to vehicle-treated OGD (Figure 7 and Figure 8, right panels).

### Example 11 - Poly-ICLC preconditioning required type I IFN signaling in vivo

To determine whether type I IFN signaling was involved in poly-ICLC neuroprotection in an intact animal, wild type and IFNAR-/- mice were treated with poly-ICLC or vehicle 72 hours prior to middle cerebral artery occlusion (n=7-10/group), (Fig. 9). Infarct volume was measured at 24 hours following middle cerebral artery occlusion. The results showed that WT mice were significantly protected by poly-ICLC preconditioning treatment, but IFNAR-/- mice were not protected (WT vehicle $34.7 \pm 3.1$ vs poly-ICLC $13.8 \pm 2.2$; IFNAR-/- $31.5 \pm 6.9$ vs poly-ICLC $35.4 \pm 4.9$ $p > 0.05$; Fig. 9).

### Claims

1. A composition for use in a method of protecting a subject at risk of developing a hypoxic injury from said hypoxic injury, the composition comprising:

   poly-ICLC in a pharmaceutically acceptable carrier, wherein the composition is formulated to be administered systemically.

2. The composition for use according to claim 1 wherein the poly-ICLC has a molecular weight of greater than 10,000 kDa.

3. The composition for use according to claim 2 wherein the poly-ICLC has a molecular weight of greater than 27,000 kDa.

4. The composition for use according to claim 1 wherein the composition is formulated for a route of administration selected from the group consisting of injection, oral, sublingual, rectal, transdermal, intranasal, vaginal, retro-orbital, and inhalation.

5. The composition for use according to claim 4 wherein the composition is formulated for injection and wherein the composition is further formulated for one or more of subcutaneous, intramuscular, intradermal, intraperitoneal, or intravenous injection.

6. The composition for use according to claim 1 wherein the hypoxic injury to which the subject is at risk results from ischemia.

7. The composition for use according to claim 6 wherein the hypoxic injury to which the subject is at risk is at risk of occurring in the brain.

8. The composition for use according to claim 7 wherein the subject is at risk of developing a hypoxic injury due to an impending surgical procedure.

9. The composition for use according to claim 8 wherein the surgical procedure is selected from the group consisting of endarterectomy, pulmonary bypass surgery, coronary artery bypass surgery, and abdominal surgery.

10. The composition for use according to claim 7 wherein the subject is at risk of developing a hypoxic injury due to a heightened risk of stroke.

**Patentansprüche**

1. Eine Zusammensetzung für die Verwendung in einem Verfahren zum Schutz eines Patienten, bei dem die Gefahr der Entwicklung einer hypoxischen Läsion besteht, von dieser hypoxischen Läsion, das Verfahren weist dabei Folgendes auf:

   Poly-ICLC auf einem pharmazeutisch zulässigen Träger, wobei die Zusammensetzung so aufgebaut ist, dass sie systemisch verabreicht werden kann.

2. Die Zusammensetzung für die Verwendung gemäß Anspruch 1, wobei die Poly-ICLC ein Molekulargewicht größer als 10.000 kDa hat.

3. Die Zusammensetzung für die Verwendung gemäß Anspruch 2, wobei die Poly-ICLC ein Molekulargewicht größer als 27.000 kDa hat.

4. Die Zusammensetzung für die Verwendung gemäß Anspruch 1, wobei die Zusammensetzung für einen Verabreichungsweg vorgesehen ist, ausgewählt aus der Gruppe bestehend aus Injektion, oral, sublingual, rektal, transdermal, intranasal, vaginal, retroorbital und Inhalation.

5. Die Zusammensetzung für die Verwendung gemäß Anspruch 4, wobei die Zusammensetzung für eine Injektion vorgesehen ist und die Zusammensetzung darüberhinaus für eine oder mehrere Formen der subkutanen, intramuskulären, intradermalen, intraperitonealen oder intravenösen Injektion vorgesehen ist.

6. Die Zusammensetzung für die Verwendung gemäß Anspruch 1, wobei sich die hypoxische Läsion, durch die der Patient gefährdet ist, aus einer Ischämie ergibt.

7. Die Zusammensetzung für die Verwendung gemäß Anspruch 6, wobei bei der hypoxischen Läsion, durch die der Patient gefährdet ist, die Gefahr besteht, dass sie im Gehirn entsteht.

8. Die Zusammensetzung für die Verwendung gemäß Anspruch 7, wobei für den Patienten, aufgrund eines bevorstehenden operativen Eingriffs, die Gefahr der Entwicklung einer hypoxischen Läsion besteht.

9. Die Zusammensetzung für die Verwendung gemäß Anspruch 8, wobei der operative Eingriff ausgewählt ist aus der Gruppe bestehend aus Endarteriektomie, pulmonaler Bypass-Operation, Koronararterien-Bypass-Operation und Bauchchirurgie.

10. Die Zusammensetzung für die Verwendung gemäß Anspruch 7, wobei für den Patienten, aufgrund eines erhöhten Infarktrisikos, die Gefahr der Entwicklung einer hypoxischen Läsion besteht.

**Revendications**

1. Une composition destinée à une utilisation dans un procédé de protection d'un sujet présentant un risque de développement d'une lésion hypoxique de ladite lésion hypoxique, la composition contenant :

   poly-ICLC dans un excipient pharmaceutiquement acceptable, où la composition est formulée de façon à être administrée de manière systémique.

2. La composition destinée à une utilisation selon la Revendication 1 où le poly-ICLC possède un poids moléculaire supérieur à 10 000 kDa.

3. La composition destinée à une utilisation selon la Revendication 2 où le poly-ICLC possède un poids moléculaire supérieur à 27 000 kDa.

4. La composition destinée à une utilisation selon la Revendication 1 où la composition est formulée pour une voie d'administration sélectionnée dans le groupe se composant d'injection, voie orale, voie sublinguale, voie rectale, voie transdermique, voie intranasale, voie vaginale, voie rétro-orbitale et inhalation.

5. La composition destinée à une utilisation selon la Revendication 4 où la composition est formulée pour une injection et où la composition est formulée en outre pour une ou plusieurs injections parmi injection sous-cutanée, intramusculaire, intradermique, intrapéritonéale ou intraveineuse.

6. La composition destinée à une utilisation selon la Revendication 1 où la lésion hypoxique pour laquelle le sujet présente un risque résulte d'une ischémie.

7. La composition destinée à une utilisation selon la Revendication 6 où la lésion hypoxique pour laquelle le sujet présente un risque présente un risque de se produire dans le cerveau.

8. La composition destinée à une utilisation selon la Revendication 7 où le sujet présente un risque de développement d'une lésion hypoxique due à une procédure chirurgicale imminente.

9. La composition destinée à une utilisation selon la Revendication 8 où la procédure chirurgicale est sélectionnée dans le groupe se composant d'endartériectomie, chirurgie de pontage pulmonaire, chirurgie de pontage coronarien et chirurgie abdominale.

10. La composition destinée à une utilisation selon la Revendication 7 où le sujet présente un risque de développement d'une lésion hypoxique due à un risque accrue d'attaque vasculaire cérébrale.

Figure 1A

Figure 1B

Figure 1C

Figure 1D

Figure 1E

Figure 2A

Figure 2B

Figure 2C

Figure 2D

**TNFα**

Figure 2E

**IFNγ**

Figure 3A

Figure 3B

Figure 3C

Figure 4A

Figure 4B

Figure 5A

Figure 5B

Figure 7

Figure 8

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4349538 A
- WO 2005102278 A, Salazar
- US 5585103 A
- US 5709860 A
- US 5270202 A
- US 5695770 A
- US 5188837 A
- US 4235871 A
- US 4501728 A
- US 4837028 A
- US 4957735 A
- US 5019369 A
- US 5055303 A
- US 5514670 A
- US 5413797 A
- US 5268164 A
- US 5004697 A
- US 4902505 A
- US 5506206 A
- US 5271961 A
- US 5254342 A
- US 5534496 A
- US 6468558 B

### Non-patent literature cited in the description

- **BARBER PA et al.** *Stroke,* 2008, vol. 39, 1427-1433
- **FUKADA J et al.** *Surgery Today,* 2004, vol. 34, 11-15
- **MEHTA RH et al.** *The American Journal of Cardiology,* 2010, vol. 106, 1728-1734
- **DIRNAGL U et al.** *Trends in Neuroscience,* 2003, vol. 26, 248-254
- **HEEMANN U.** *American Journal of Pathology,* 2000, vol. 156, 287-293
- **HUA F et al.** *Journal of Neuroimmunology,* 2008, vol. 199, 75-82
- **ROSENZWEIG HL et al.** *J Cereb Blood Flow & Metab,* 2007, vol. 27, 1663-1674
- **STEVENS SL et al.** *J Cereb Blood Flow Metab,* 2008, vol. 28, 1040-1047
- **TASAKI et al.** *Brain Research,* 1997, vol. 748, 267-270
- **BENJAMIN LEWIN.** Genes V. Oxford University Press, 1994
- The Encyclopedia of Molecular Biology. Blackwell Science Ltd, 1994
- Molecular Biology and Biotechnology: a Comprehensive Desk Reference. VCH Publishers, Inc, 1995
- Remingtons Pharmaceutical Sciences. Mack Publishing Company, 1995
- **FIELD et al.** *PNAS USA,* 1967, vol. 58, 1004-1010
- **LEVY et al.** *PNAS USA,* 1969, vol. 62, 357-361
- **MARKOSIAN M ; HYDE RM.** *Antiviral Chemistry and Chemotherapy,* 2005, vol. 16, 91-102
- **ROSENFELD MR et al.** *Neuro-oncology,* 2010, vol. 12, 1071-1077
- Remington's Pharmaceutical Sciences. Mack Publishing Company, 1995
- **SCHMOLKA.** *J. Am. Oil. Chem. Soc.,* 1977, vol. 54, 110
- **HUNTER et al.** *J. Immunol.,* vol. 129, 1244, , 1981
- **HUNTER ; BENNETT.** *J. Immun.,* 1984, vol. 133, 3167
- **LANGER.** *Accounts Chem. Res.,* 1993, vol. 26, 537
- **JOHNSTON et al.** *Pharm. Res.,* 1992, vol. 9, 425
- **PEC.** *J. Parent. Sci. Tech.,* 1990, vol. 44 (2), 58
- **IJNTEMA et al.** *Int. J. Pharm.,* 1994, vol. 112, 215
- **BETAGERI et al.** Liposome Drug Delivery Systems. Technomic Publishing Co., Inc, 1993
- **CLARK WM et al.** *Neurol Res,* 1997, vol. 19, 641-648
- **ZHANG L et al.** *J Neurosci Methods,* 2002, vol. 117, 207-214
- **KIS B et al.** *Neuroreport,* 2001, vol. 12, 4139-4142
- **DELI MA et al.** *Cell Mole Neurobiol,* 2003, vol. 25, 59-127
- **PERRIERE N et al.** *J Neurochem,* 2005, vol. 93, 279-289
- **DELI et al.** *Inflamm Res,* 2003, vol. 52 (1), S39-S40
- **RUBIN LL et al.** *J Cell Biol,* 1991, vol. 115, 1725-1735

- **GESUETE et al.** *Stroke,* 2011, vol. 42, 1445-1453
- **LEVY HB et al.** *J Infect Dis,* 1975, 434-439
- **LEVY HB et al.** *Tex Rep Biol Med,* 1981, vol. 41, 653-662
- **BEVER CT et al.** *J Interferon Res,* 1985, vol. 5, 423-428
- **BEVER CT et al.** *J Interferon Res,* 1988, vol. 8, 419-425
- **LONGHI et al.** *J Exp Med,* 2009, vol. 206, 1589-1602